# EUROPÄISCHE PATENTSCHRIFT

(11) **EP 0 449 778 B1**
(45) Veröffentlichungstag und Bekanntmachung des Hinweises auf die Patenterteilung: **14.09.1994**
(21) Anmeldenummer: 91810194.0
(22) Anmeldetag: 21.03.1991
(51) Int. Cl.: C07C 69/732, C07C 67/347

(54) **Verfahren zur Herstellung von Hydroxyphenylpropionaten**
Process for the preparation of hydroxyphenylepropionates
Procédé de préparation d'hydroxyphénylpropionates

(30) Priorität: 30.03.1990 CH 1058/90
(43) Veröffentlichungstag der Anmeldung: 02.10.1991
(73) Patentinhaber: CIBA-GEIGY AG, 4002 Basel (CH)
(72) Erfinder: Kovàsy, Kàlmàn, Dr., CH-4103 Bottmingen (CH); Mazour, Zdenek, CH-4415 Lausen (CH)

(56) Entgegenhaltungen:
- WO-A-86/00301

## Beschreibung

Die vorliegende Erfindung betrifft ein neues Verfahren zur Herstellung von Hydroxyphenylpropionaten.

Hydroxyphenylpropionate werden meist durch sogenannte Michael-Addition aus einem Phenol und einem Acrylsäurealkylester in Gegenwart eines basischen Katalysators hergestellt. Dies ist beispielsweise in DE-A-3390557 gezeigt. Die Additionsprodukte, also Hydroxyphenylpropionate, können dann durch Kristallisation oder, wie in US-A-3,330,859 und US-A-3,364,250 ausgeführt, auch durch Destillation von Nebenprodukten befreit werden.

Die so erhaltenen Hydroxyphenylpropionate sind wichtige Zwischenprodukte für die Herstellung von Antioxidantien für synthetische Polymere, insbesondere Polyolefine, wie sie z.B. in obigen Dokumenten beschrieben sind. Durch Verändern des Molekulargewichts der Estergruppe kann man bestimmten Anforderungen, die an ein Antioxidans gestellt werden, wie z.B. geringe Flüchtigkeit, gerecht werden. Dies ist direkt durch die Synthese, aber auch indirekt, beispielsweise durch Umesterung eines bereits hergestellten Hydroxyphenylpropionats, erreichbar.

Es hat sich nun gezeigt, dass durch eine Kombination von bestimmten Massnahmen, nämlich Neutralisation des basischen Katalysators durch eine Carbonsäure, Entfernung des gebildeten Salzes aus der Reaktionsmasse und anschliessende Rektifikation des Filtrats das Verfahren insbesondere hinsichtlich Ausbeute und Reinheit des Endproduktes deutlich verbessert werden kann.

Gegenstand der vorliegenden Erfindung ist somit ein Verfahren zur Herstellung von Verbindungen der Formel 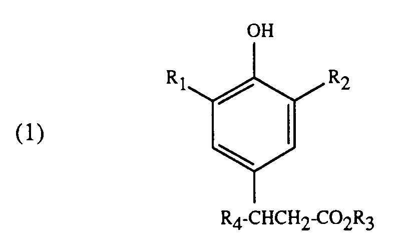
durch Umsetzung von Verbindungen der Formel 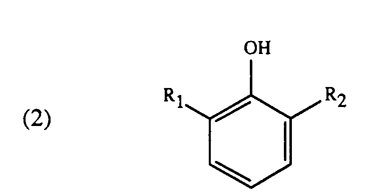
mit Verbindungen der Formel

(3) R₄-CH=CH₂-CO₂R₃,

worin in den Formeln (1) bis (3) die Substituenten R₁ bis R₃ unabhängig voneinander Alkyl mit 1 bis 4 Kohlenstoffatomen sind, und R₄ Wasserstoff oder Alkyl mit 1 bis 4 Kohlenstoffatomen ist, in Gegenwart einer Base und Isolierung der Verbindungen der Formel (1), dadurch gekennzeichnet, dass man nach der Umsetzung einer Verbindung der Formel (2) mit einer Verbindung der Formel (3) und vor der Isolierung einer Verbindung der Formel (1) die Reaktionsmasse zur Neutralisation der Base mit einer Carbonsäure versetzt, die Reaktionsmasse filtriert und zur Isolierung der Verbindung der Formel (1) rektifiziert.

In den Verbindungen der Formel (1) bedeuten die Substituenten R₁ bis R₃ unabhängig voneinander Alkyl mit 1 bis 4 Koblenstoffatomen, beispielsweise Methyl, Aethyl, Propyl, Butyl oder t-Butyl. Der Substituent R₄ hat dieselbe Bedeutung wie R₁, R₂ oder R₃, kann aber zusätzlich auch Wasserstoff bedeuten.

Als Base kann prinzipiell jede Verbindung eingesetzt werden, deren Basizität höher ist als die des eingesetzten Phenols. Im allgemeinen werden jedoch meist Alkoholate, insbesondere Alkalimetallalkoholate, insbesondere solche mit 1-4 C-Atomen, wie Natrium- oder Kaliummethanolat oder -t-butylat verwendet. Alkalimetallhydroxide wie Natrium- oder Kaliumhydroxid bzw. das mit ihnen aus dem Phenol der Formel (2) gebildete Phenolat können ebenfalls die Rolle des Katalysators übernehmen.

Bis zur Stufe der Neutralisation der Base kann das erfindungsgemässe Verfahren auf bekannte, z.B. in der genannten Literatur beschriebene Weise durchgeführt werden. Es kann sich als vorteilhaft erweisen, nach der Zugabe der Verbindung der Formel (3) und vor der Neutralisation dem Reaktionsgemisch noch Wasser zuzusetzen. Dies ist insbesondere dann der Fall, wenn Verbindungen der Formel (2) umgesetzt werden sollen, worin einer der Substituenten R₁ oder R₂ beispielsweise Methyl bedeutet. Vorzugsweise fügt man dann 0,1 bis 1 % Wasser (bezogen auf das Gewicht von zugesetzter Verbindung der Formel (3)) hinzu.

Erfindungsgemäss wird die Base mit einer Carbonsäure neutralisiert. Vorzugsweise handelt es sich dabei um Ameisen- oder Essigsäure. Zur Abtrennung des Formiats oder Acetats filtriert man die Reaktionsmasse auf übliche Weise. Es wurde nämlich gefunden, dass diese Salze die Durchführung der anschliessenden Rektifikation beeinträchtigen können und bei den für die Rektifikation notwendigen Temperaturen die Abspaltung der Substituenten R₁ und R₂ begünstigen können. Vorzugsweise neutralisiert und filtriert man bei einer Temperatur von 50 bis 130°C, insbesondere 70 bis 110°C.

Die zugesetzte Menge an Carbonsäure entspricht zweckmässig mindestens der äquivalenten Menge an vorhandener Base; vorzugsweise wird ein Ueberschuss an Säure, z.B. bis zu 50 %, eingesetzt.

Zur Isolierung der Verbindungen der Formel (1) bedient man sich bevorzugt zweier kontinuierlich arbeitender Rektifizierkolonnen, die bei reduziertem Druck betrieben werden. In der ersten Kolonne werden leichterflüchtige Nebenkomponenten als Kopfprodukt und entsprechend in der anderen Kolonne die schwererflüchtigen Nebenkomponenten als Sumpfprodukt abgetrennt. Die gereinigte Verbindung der Formel (1) fällt in der zweiten Kolonne an, und zwar als Kopfprodukt.

Es ist auch möglich, in der ersten Kolonne die schwererflüchtigen Nebenkomponenten abzutrennen. Dann werden in der zweiten Kolonne die leichterflüchtigen Nebenkomponenten entfernt, wonach die gereinigte Verbindung der Formel (1) als Sumpfprodukt anfällt.

Als Alternative zur Verwendung zweier kontinuierlich arbeitender Rektifizierkolonnen kann auch eine einzige kontinuierlich arbeitende Rektifizierkolonne eingesetzt werden, die eine Seitenentnahemstelle besitzt. In diesem Fall wird die Rektifikation so geführt, dass leichterflüchtige Nebenkomponenten als Kopfprodukt und die schwererflüchtigen Komponenten als Sumpfprodukt anfallen, während die gereinigte Verbindung der Formel (1) durch eine Seitenentnahmstelle abgeführt wird. Liegt die Seitenentnahmestelle zwischen Speisung und Sumpf, so wird die Verbindung der Formel (1) aus der Gasphase entnommen. Aus der flüssigen Phase wird sie entnommen, wenn die Seitenentnahme zwischen Kopf und Speisung liegt.

Als erste der beiden kontinuierlich arbeitenden Rektifizierkolonnen verwendet man vorzugsweise eine Vakuumkolonne, welche sowohl einen Abtriebsteil als auch einen Verstärkerteil hat. Als Kopfprodukt werden hier u.a. das überschüssige Phenol und das entsprechende Chinon (meistens eine farbige Komponente) abgetrennt. Es ist zweckmässig, einen sehr tiefen Kopfdruck zu wählen (z.B. 3 mbar) und auch den Druckabfall über die Packung klein zu halten, damit die thermische Schädigung klein bleibt. Das erforderliche Rücklaufverhältnis hängt weitgehend von Art und Menge des abzutrennenden Phenols ab.

In der zweiten Rektifizierkolonne, welche aus denselben Gründen zweckmässig bei ähnlich tiefen Drücken arbeitet wie die erste Kolonne, werden die schwererflüchtigen Verunreinigungen vom Produkt getrennt. Das gereinigte Produkt fällt als Destillat an. Das erforderliche Rücklaufverhältnis ist meistens nicht gross (z.B. 1:1), und oft kann man auch auf eine Abtriebskolonne verzichten, ohne dass dadurch merkliche Mengen des Produktes in den Sumpfablauf gelangen würden.

Vorzugsweise schaltet man den genannten Rektifizierkolonnen einen Verdampfer voran, der bei höherem Druck als diese Kolonnen arbeitet. Es können übliche Verdampfer wie Fallfilmverdampfer und Dünnschichtverdampfer verwendet werden. Es lassen sich auch zwei solche Verdampfer hintereinanderschalten. Zweck dieser Verdampfer ist es, ganz leicht siedende Komponenten wie Wasser, Carbonsäure und Acrylsäureester vor der Rektifikation bis auf einen minimalen, tolerierbaren Restgehalt auszudampfen (Entgasung).

Die Verdampfer arbeiten vorzugsweise bei einem Druck von 20 bis 200 mbar, während der Kopfdruck in den Rektifizierkolonnen vorzugsweise zwischen 1 und 30 mbar liegt.

Das aus der Entgasung kommende Rohgemisch kann beim Druck, der an der Zulaufstelle in der Kolonne herrscht, überhitzt sein, so dass hier eine Entspannungsverdampfung stattfindet. Bei der Auslegung der Kolonnen muss dieser Umstand entsprechend berücksichtigt werden.

Erfindungsgemäss kann die neutralisierte und filtrierte Reaktionsmasse auch absatzweise rektifiziert werden. Man benötigt dazu eine gegebenenfalls gerührte Destillierblase, eine Kolonne mit einer effizienten Vakuumpackung, Kondensator, Rücklaufteiler und eine Anzahl Behälter für die verschiedenen Fraktionen. Eine Trennleistung von zehn theoretischen Stufen ist in der Regel hinreichend, aber nicht unbedingt erforderlich. Nachstehend sind beispielhaft einzelne Fraktionen aufgeführt mit den dazugehörigen Richtwerten von Rücklaufverhältnis und Kopfdruck:

| | Rücklaufverhältnis | Kopfdruck (mbar) |
|---|---|---|
| 1. Fraktion (sehr leichtflüchtige Komponenten): | 0 | 20-200 |
| 2. Fraktion (u.a. Phenole): | ≧ 2:1 | 1-30 |
| 3. Fraktion (Phenole und Verbindungen der Formel (1)) | ≧ 2:1 | 1-30 |
| 4. Fraktion (gereinigte Verbindungen der Formel (1)) | ≦ 1:1 | 1-30 |
| 5. Fraktion (Produkt und Schwererflüchtige): | ≦ 1:1 | 1-30 |

Die erste Fraktion entspricht der Entgasung bei der kontinuierlichen Rektifikation. Wenn ihre Menge sehr klein ist, gelangt sie gar nicht in den Kondensator, sondern bleibt an der Kolonnenpackung hängen. Sie entweicht dann in die Vakuumpumpe, wenn bei der zweiten Fraktion der Druck abgesenkt wird. Die zweite Fraktion enthalt die Phenole. Sie wird als Abfall betrachtet. Das Rücklaufverhältnis hängt weitgehend davon ab, wieviel Produkt man in dieser Fraktion tolerieren will. Die dritte und die Fünfte Fraktion sind Zwischenfraktionen, die man zweckmässigerweise dem nächsten Ansatz beimischt. Sollten sich in ihnen mit der Zeit unerwünschte Nebenkomponenten anreichern, so muss man sie von Zeit zu Zeit teilweise oder ganz verwerfen.

Vorzugsweise werden nach dem erfindungsgemässen Verfahren solche Verbindungen der Formel (1) hergestellt, worin R₁ t-Butyl, R₂ Methyl oder t-Butyl, R₄ Wasserstoff und R₃ Methyl ist. Die Base wird nach der Michael-Addition mit Ameisen- oder Essigsäure neutralisiert. Die Reaktionsmasse filtriert man dann und trennt zur Isolierung der Verbindung der Formel (1) in einer Rektifizieranlage auf, die aus zwei kontinuierlich arbeitenden Rektifizierkolonnen und einem vorangeschalteten Verdampfer besteht, wobei die erste der beiden Rektifizierkolonnen sowohl einen Abtriebs- als auch einen Verstärkerteil, die zweite dieser Kolonnen aber nur einen Verstärkerteil aufweist.

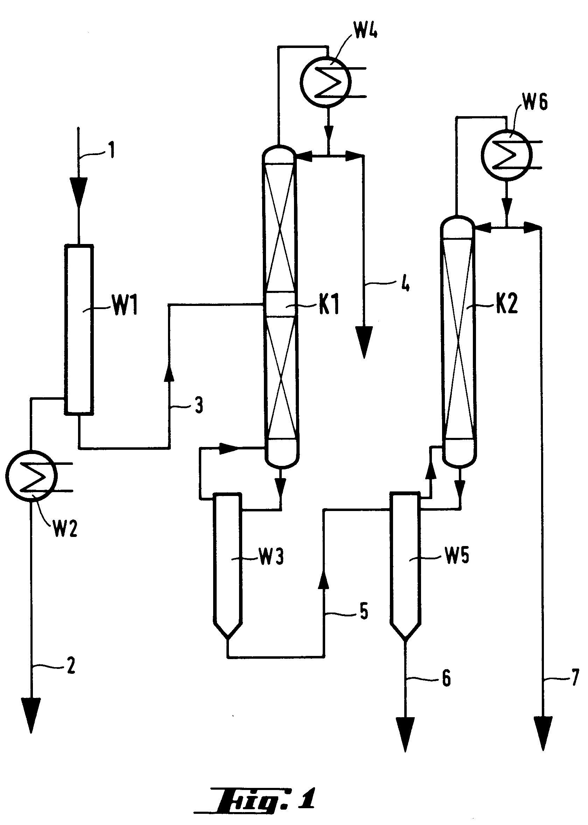

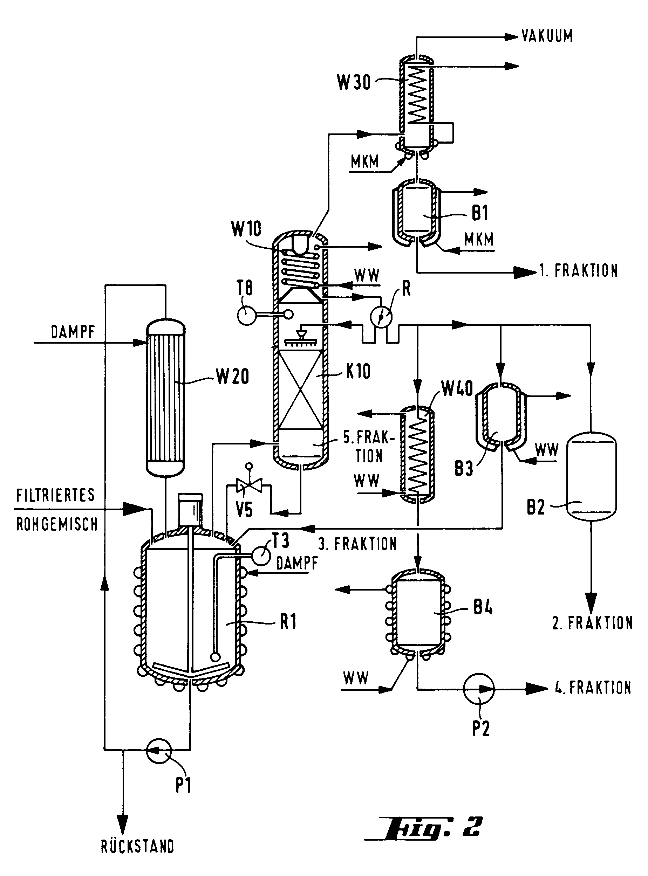

In den Figuren 1 und 2 sind schematisch Anlagen für die kontinuierliche und absatzweise durchgeführte Rektifikation dargestellt.

In Figur 1 bedeuten die mit den Ziffern 1 bis 7 gekennzeichneten Linien Massenströme, wobei 1 den Massenstrom des Rohprodukts und 7 den Massenstrom des Reinprodukts darstellt. Die Massenströme 2, 4 und 6 verdeutlichen die Abtrennung von Nebenprodukten. 3 und 5 zeigen die stufenweise Reinigung des Rohprodukts. K1 und K2 sind Rektifizierkolonnen, wobei K1 einen Verstärker- und Abtriebsteil, K2 nur einen Verstärkerteil besitzt. W1, W3 und W5 sind Verdampfer, während W2, W4 und W6 als Kondensator arbeiten. In Beispiel 1 ist die kontinuierliche Arbeitsweise der in dieser Figur gezeigten Anlage näher beschrieben.

Die absatzweise durchgeführte Rektifikation des Rohprodukts ist in Figur 2 dargestellt. Der Rührkessel R1 steht in Verbindung mit der Rektifizierkolonne K10 mit aufgesetztem Kondensator W10. W30 und W40 stellen einen Kühler dar, W20 arbeitet als Verdampfer. Zum Auffangen verschiedener Fraktionen dienen die Behälter B1 bis B4. R ist ein Rücklaufteiler, P1 und P2 stellen Pumpen dar. Eine genaue Beschreibung der Arbeitsweise dieser Anlage ist in Beispiel 2 gegeben.

Die folgenden Beispiele erläutern die Erfindung weiter, ohne sie darauf zu beschränken. Prozentangaben beziehen sich darin sowie in den Patentansprüchen und in der übrigen Beschreibung auf das Gewicht, sofern nichts anderes angegeben ist.

Beispiel 1: 3,5-Di-tert.-butyl-4-hydroxyphenyl-1-propionsäuremethylester wird durch Anlagerung von Acrylsäuremethylester an 2,6-Di-tert.-butylphenol hergestellt. Dabei wird zuerst eine Schmelze von 600 g 2,6-Di-tert.-butylphenol mit 6,5 g wässriger, 50 %iger Kalilauge unter Stickstoffatmosphäre versetzt, wobei sich Kaliumphenolat bildet. Danach wird das Wasser bei einer Temperatur von 100°C und einem Druck von 20 mbar abdestilliert und die Reaktion mit 325 g Acrylsäuremethylester bei ca. 110°C und Normaldruck durchgeführt. Der überschüssige Acrylsäuremethylester wird im Vakuum (10 mbar) bei 115°C abdestilliert und das Reaktionsgemisch nach Kühlen auf 90°C mit 2,8 g Ameisensäure (100 %) versetzt. Das ausgefallene Kaliumformiat wird dann bei 90°C durch Filtration entfernt. Man erhält so ein Rohgemisch des 3,5-Di-tert.-butyl-4-hydroxyphenyl-1-propionsäuremethylester.

Das so hergestellte Rohgemisch des 3,5-Di-tert.-butyl-4-hydroxyphenyl-1-propionsäuremethylester (Filtrat) wird in einer kontinuierlich arbeitenden Laborrektifizieranlage, die in Fig. 1 schematisch dargestellt ist, gereinigt. Das Rohgemisch wird mit einem Massestrom von 1212 g/h in den Fallfilmverdampfer W1 gespeist (Strom 1), wo es bei 103 Torr auf 185°C aufgeheizt und dabei entgast wird. Die entweichenden Dämpfe werden im Kondensator W2 kondensiert (Strom 2). Das entgaste Rohgemisch wird in die Kolonne K1 geleitet (Strom 3) und dabei in der Leitung auf ca. 115°C abgekühlt. Dadurch wird eine Entspannungsverdampfung beim Eintritt in die Kolonne K1 vermieden. Diese arbeitet bei einem Kopfdruck von 2,6 Torr, wobei der Druck im Sumpf ca. 7,2 Torr beträgt. Die Brüden werden im Kondensator W4 kondensiert und zum Teil entnommen, zum Teil in die Kolonne zurückgeführt. Mit dem Rücklaufverhältnis wird eine ungefähr in der Mitte der Verstärkerkolonne gemessene Temperatur geregelt. Das Rücklaufverhältnis ist so gewissen Schwankungen ausgesetzt, ist aber immer grösser als 10:1. In K1 werden die leichterflüchtigen Nebenkomponenten als Kopfprodukt abgetrennt (Strom 4). Als Verdampfer benützt man einen Dünnschichtverdampfer (W3). Die Temperatur beträgt im Kopf ca. 96°C und im Sumpf ca. 181°C. Das Gemisch gelangt aus dem Sumpf der Kolonne K1 in den Verdampfer W5 der Kolonne K2 (Strom 5). Dieser ist ebenfalls ein Dünnschichtverdampfer. Die Brüden der Kolonne werden im Kondensator W6 kondensiert, ein Teil davon als gereinigtes Produkt entnommen (Strom 7) und der Rest in die Kolonne zurückgeführt. Das Rücklaufverhältnis beträgt bei Kolonne K2 1:1. Die schwererflüchtigen Nebenkomponenten verlassen die Kolonne als Sumpfprodukt (Strom 6). K2 arbeitet bei einem Kopfdruck von 2,6 Torr, wobei der Sumpfdruck ca. 6,2 Torr betragt. Die Temperatur beträgt im Kopf ca. 163°C und unterhalb der Kolonnenpackung ca. 186°C.

Das Rohgemisch (Strom 1) enthält 94,3 % 3,5-Di-tert.-butyl-4-hydroxyphenyl-1-propionsäuremethylester, 0,9 % 2,6-Di-tert.-butylphenol, 0,8 % 2,4-Di-tert.-butylphenol, Spuren von Acrylsäuremethylester, Ameisensäure und Wasser, sowie grösstenteils unbekannte und zum Teil färbende Nebenkomponenten. Das gereinigte Produkt (Strom 7) enthält 99,5 % 3,5-Di-tert.-butyl-4-hydroxyphenyl-1-propionsäuremethylester, keine Phenole und keine färbenden Nebenkomponenten. Die Phenole bleiben im Strom 4 und die färbenden Nebenkomponenten teils in Strom 4, teils in Strom 6. Mehr als 99 % des im Strom 1 vorhandenen 3,5-Di-tert.-butyl-4-hydroxyphenyl-1-propionsäuremethylester werden im Strom 7 wieder gefunden.

Alle Apparaturen sind aus Glas, nur gewisse Teile aus rostfreiem Stahl (z.B. Rotorwellen der Dünnschichtverdampfer W3 und W5). Die Kolonnen K1 und K2 haben einen Innendurchmesser von 50 mm und waren vakuumisoliert. Die Packung besteht aus 8 x 8 mm Maschendrahtringen mit Steg (Werkstoff: rostfreier Stahl). Die Packungshöhen betragen bei K1 im Verstärkerteil 32 cm und im Abtriebsteil 32 cm, bei K2 im Verstärkerteil 45 cm.

Die Dünnschichtverdampfer W3 und W5 haben eine Heizfläche von 0,06 m² und der Fallfilmverdampfer W1 hat eine Heizfläche von ähnlicher Grösse.

Beispiel 2: 3-Methyl-5-tert.-butyl-4-hydroxyphenyl-1-propionsäuremethylester wird durch Anlagerung von Acrylsäuremethylester an 2-Methyl-6-tert.-butylphenol hergestellt. Die Herstellung erfolgt analog zu dem in Beispiel 1 beschriebenen Verfahren, wobei jedoch 3 Stunden nach Zugabe des Acrylsäuremethylesters ein Zusatz von 0,4 % Wasser (bezogen auf eingesetzten Acrylsäuremethylester) erfolgt.

Nach der Reaktion wird der überschüssige Acrylsäuremethylester in Vakuum abdestilliert, wobei die (Wand-) Temperatur 115°C nicht übersteigen soll, und das Reaktionsgemisch mit Ameisensäure neutralisiert. Das ausgefallene Kaliumformiat wird bei 90°C durch Filtration abgetrennt. Danach erfolgt die Reinigung des Produktes durch Rektifikation.

In einer Produktionsanlage wird die Synthese absatzweise in einem 6,3 m³-Rektor durchgeführt. Zur Filtration wird ein Schraubenfilter eingesetzt (Filterfläche: 12,6 m²), an welchem die Filtration einer Partie in höchstens einer Stunde durchgeführt wird. Das filtrierte Rohgemisch wird absatzweise rektifiziert. Die Rektifizierapparatur ist schematisch in Fig. 2 dargestellt. Sie besteht aus einem Rührkessel R1, aus einer Rektifizierkolonne K10 mit aufgesetztem Spiralkondensator W10 und externem Rücklaufteiler R, aus einem als Kühlfalle benutzten Spiralschlangen-Kondensator W30, aus einem als Destillatkühler benutzten Spiralschlangen-Rieselkühler W40 und aus einem Fallfilmverdampfer W20, über welchen der Inhalt des Rührkessels mit Pumpe P1 umgewälzt wird. W20 dient zur Erhöhung der Verdampferleistung. Die verschiedenen Fraktionen werden in den Behältern B1 bis B4 aufgefangen. Alle Apparate sind aus rostfreiem Stahl. Ihre charakteristischen Grössen sind die folgenden (F = Wärmeaustauschfläche, V = Nennvolumen):

| | |
|---|---|
| R1: | V = 6,3 m³ |
| W10: | F = 16 m² |
| W20: | F = 32 m² |
| W30: | F = 2,5 m² |
| B1: | V = 0,1 m³ |
| B2: | V = 0,63 m³ |
| B3: | V = 0,25 m³ |
| B4: | V = 0,63 m³ |
| K10: | Durchmesser: 1,2 m |
| | Packungshöhe: 2,73 m |
| | Packung: Mellapak 350 Y (Sulzer) |

Bei den Apparaten W10, W40, B3 und B4 wird Warmwasser (WW) als Kühl- bzw. Heizmedium eingesetzt. Es muss eine Temperatur von ca. 50°C haben, da das 3-Methyl-5-tert.-butyl-4-hydroxyphenyl-1-propionsäuremethylester einen Schmelzpunkt von 44,5°C besitzt. W30 und B1 werden mit Methanol (MKM) gekühlt, welches eine Temperatur von ca. -35°C besitzt.

Eine filtrierte Partie enthält ca. 84,3 % 3-Methyl-5-tert.-butyl-4-hydroxyphenyl-1-propionsäuremethylester, 4,1 % 3-Methyl-5-tert.-butylphenol, 0,1 bis 0,2 % Ameisensäure, Spuren von Acrylsäuremethylester und eventuelle Spuren von Wasser. Der Rest besteht vorwiegend aus schwererflüchtigen Nebenkomponenten. Vor Beginn der Rektifikation wird noch die Zwischenfraktion und die Endfraktion aus der vorangehenden Partie beigemischt (Fraktion 3 und 5). Zuerst wird der Kesselinhalt bei totalem Rücklauf auf 155°C aufgeheizt und die Apparatur gleichzeitig auf 20 mbar evakuiert. Dabei entweichen die sehr leichtflüchtigen Komponenten wie Acrylsäuremethylester, Ameisensäure und Wasser in die Kühlfalle W30 und werden als Kondensat im Behälter B1 gesammelt. Nach einer Haltezeit von ca. 15 Min. wird das Kondensat aus B1 ausgeschleust (1. Fraktion). Danach wird der Kopfdruck auf 4 mbar gesenkt und der Kesselinhalt sukzessive aufgeheizt, bis totaler Rücklauf eintritt. Dann wird die 2. Fraktion mit einem Rücklaufverhältnis von 1:1 entnommen und in B2 gesammelt (Phenolfraktion). Sie ist beendet, wenn die Kopftemperatur einen Wert von T8 ≈ 110°C erreicht hat. Die 3. Fraktion wird mit einem Rücklaufverhältnis von 3:1 entnommen und in B3 gesammelt. Sie ist beendet, wenn die Kopftemperatur einen Wert von T8 ≈ 160°C erreicht hat (Zwischenfraktion). Diese Grenztemperaturen sind nur Richtwerte, die bei der Inbetriebnahme optimiert werden müssen. Die 4. Fraktion (gereinigtes Produkt) wird ohne Rücklauf entnommen und in Behälter B4 aufgefangen, der von Zeit zu Zeit mit Pumpe P2 leergepumpt wird. Diese Fraktion ist beendet, wenn die Innentemperatur den Wert von T3 ≈ 192°C erreicht hat. Dann wird die Kolonne auf totalen Rücklauf gestellt, das Ablaufventil V5 geschlossen und die 5. Fraktion im Unterteil der Kolonne aufgefangen (Endfraktion). Sie ist beendet, wenn die Innentemperatur den Wert von T3 ≈ 196°C erreicht hat. Diese Grenztemperaturen sind wiederum nur Richtwerte, die bei der Inbetriebnahme optimiert werden müssen.

Bei einer Partiegrösse (filtriertes Rohgemisch) von 6342 kg sind folgende Mengen bei den einzelnen Fraktionen zu erwarten, wenn man am Anfang die 3. und 5. Fraktion aus der vorherigen Partie auch dazugibt:

| | |
|---|---|
| 1. Fraktion | 38 kg |
| 2. Fraktion (Phenolfrak.) | 342 kg |
| 3. Fraktion (Zwischenfrak.) | 58 kg |
| 4. Fraktion (Produkt) | 5371 kg |
| 5. Fraktion (Endfraktion) | 101 kg |
| Rückstand | 591 kg |

Die 4. Fraktion enthält ca. 97,6 % 3-Methyl-5-tert.-butyl-4-hydroxyphenyl-1-propionsäuremethylester und die Verluste an 3-Methyl-5-tert.-butyl-4-hydroxyphenyl-1-propionsäuremethylester bei der Rektifikation betragen ca 2 %. Dämpfe, die beim Evakuieren in die Vakuumpumpe entweichen, werden bei den obigen Mengenangaben zur 1. Fraktion gerechnet. Die Rektifikation einer Partie der oben angegebenen Grösse dauert einschliesslich Filtration höchstens 12 Stunden.

## Patentansprüche

1. Verfahren zur Herstellung von Verbindungen der Formel 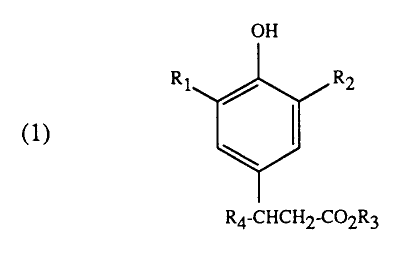 durch Umsetzung von Verbindungen der Formel 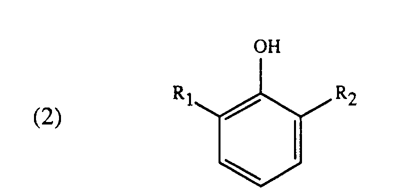 mit Verbindungen der Formel
(3) R₄-CH=CH₂-CO₂R₃,
worin in den Formeln (1) bis (3) die Substituenten R₁ bis R₃ unabhängig voneinander Alkyl mit 1 bis 4 Kohlenstoffatomen sind, und R₄ Wasserstoff oder Alkyl mit 1 bis 4 Kohlenstoffatomen ist, in Gegenwart einer Base und Isolierung der Verbindungen der Formel (1), dadurch gekennzeichnet, dass man nach der Umsetzung einer Verbindung der Formel (2) mit einer Verbindung der Formel (3) und vor der Isolierung einer Verbindung der Formel (1) die Reaktionsmasse zur Neutralisation des basischen Katalysators mit einer Carbonsäure versetzt, die Reaktionsmasse filtriert und zur Isolierung der Verbindung der Formel (1) rektifiziert.

2. Verfahren nach Anspruch 1, dadurch gekennzeichnet, dass zur Neutralisation der Base Ameisen- oder Essigsäure verwendet wird.

3. Verfahren nach Anspruch 1, dadurch gekennzeichnet, dass die Neutralisation und Filtration bei einer Temperatur von 50 bis 130°C durchgeführt wird.

4. Verfahren nach Anspruch 1, dadurch gekennzeichnet, dass man zur Isolierung der Verbindung der Formel (1) diese unter Verwendung zweier kontinuierlich arbeitender Rektifizierkolonnen unter reduziertem Druck von schwerer- und leichterflüchtigen Nebenprodukten abtrennt.

5. Verfahren nach Anspruch 1, dadurch gekennzeichnet, dass man zur Isolierung der Verbindung der Formel (1) diese unter Verwendung einer kontinuierlich arbeitenden Rektifizierkolonne mit Seitenentnahmestelle über diese Seitenentnähmestelle abtrennt.

6. Verfahren nach einem der Ansprüche 4 oder 5, dadurch gekennzeichnet, dass man den kontinuierlich arbeitenden Rektifizierkolonnen bzw. der kontinuierlich arbeitenden Rektifizierkolonne mit Seitenentnähmestelle einen Verdampfer voranschaltet, der bei höherem Druck als die Kolonnen bzw. Kolonne mit Seitenentnahmestelle arbeitet.

7. Verfahren nach einem der Ansprüche 4 oder 5, dadurch gekennzeichnet, dass der Kopfdruck der Rektifizierkolonnen bzw. der Rektifizierkolonne mit Seitenentnahmestelle 1 bis 30 mbar beträgt.

8. Verfahren nach Anspruch 6, dadurch gekennzeichnet, dass der Druck im Verdampfer 20 bis 200 mbar beträgt.

9. Verfahren nach Anspruch 1, dadurch gekennzeichnet, dass man zur Isolierung der Verbindung der Formel (1) diese unter Verwendung einer absatzweise arbeitenden Rektifizierkolonne unter reduziertem Druck von schwerer- und leichterflüchtigen Nebenprodukten abtrennt.

10. Verfahren nach Anspruch 1 zur Herstellung von Verbindungen der Formel (1), worin R₁ t-Butyl, R₂ Methyl oder t-Butyl, R₄ Wasserstoff und R₃ Methyl ist, durch Umsetzung von Verbindungen der Formel (2), worin R₁ und R₂ die angegebenen Bedeutungen haben, mit Verbindungen der Formel (3), worin R₃ und R₄ die angegebenen Bedeutungen haben, in Gegenwart einer Base, dadurch gekennzeichnet, dass man nach der Umsetzung einer Verbindung der Formel (2) mit einer Verbindung der Formel (3) und vor der Isolierung einer Verbindung der Formel (1) die Reaktionsmasse zur Neutralisation der Base mit Ameisen- oder Essigsäure vermischt, die Reaktionsmasse filtriert und zur Isolierung der Verbindung der Formel (1) aus dieser Reaktionsmasse eine Rektifizieranlage verwendet, die aus zwei kontinuierlich arbeitenden Rektifizierkolonnen und einem vorangeschalteten Verdampfer besteht, wobei die erste der beiden Rektifizierkolonnen einen Abtriebs- und einen Verstärkerteil, die zweite dieser Kolonnen aber nur einen Verstärkerteil aufweist.

11. Verfahren nach Anspruch 10 zur Herstellung von Verbindungen der Formel (1), worin R₁ t-Butyl, R₂ Methyl, R₄ Wasserstoff und R₃ Methyl ist, durch Umsetzung von Verbindungen der Formel (2), worin R₁ und R₂ die angegebenen Bedeutungen haben, mit Verbindungen der Formel (3), worin R₃ und R₄ die angegebenen Bedeutungen haben, in Gegenwart einer Base, dadurch gekennzeichnet, dass man nach der Umsetzung einer Verbindung der Formel (2) mit einer Verbindung der Formel (3) und vor der Neutralisation der Reaktionsmasse Wasser zuführt.

## Claims

1. A process for the preparation of a compound of formula 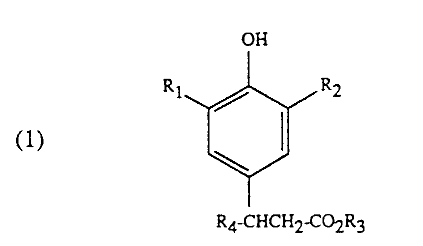 by reacting a compound of formula 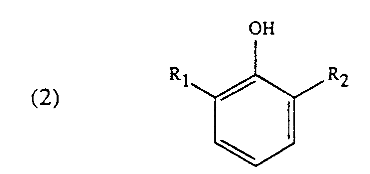 with a compound of formula
(3) R₄-CH=CH₂-CO₂R₃,
in which the substituents R₁ to R₃ in formulae (1) to (3) are each independently of one another alkyl of 1 to 4 carbon atoms, and R₄ is hydrogen or alkyl of 1 to 4 carbon atoms, in the presence of a base, and isolating the compound of formula (1), which process comprises adding a carboxylic acid to the reaction mixture to neutralize the basic catalyst after the reaction of a compound of formula (2) with a compound of formula (3) and before the isolation of a compound of formula (1), filtering the reaction mixture, and rectifying the filtrate to isolate the compound of formula (1).

2. A process according to claim 1, wherein formic acid or acetic acid is used for neutralizing the base.

3. A process according to claim 1, wherein neutralization and filtration are carried out in the temperature range from 50 to 130°C.

4. A process according to claim 1, wherein the compound of formula (1) is isolated by using two continuously operating rectifying columns, under reduced pressure, to separate less volatile and more volatile by-products from said compound.

5. A process according to claim 1, wherein the compound of formula (1) is isolated by using a continuously operating rectifying column with a side outlet to draw off said compound via said side outlet.

6. A process according to either claim 4 or claim 5, wherein an evaporator is connected upstream of the continuously operating rectifying columns or the continuously operating rectifying column with side outlet, which evaporator is operated at a pressure higher than that in the columns or column with side outlet.

7. A process according to either claim 4 or claim 5, wherein the head pressure of the rectifying columns or rectifying column with side outlet is 1 to 30 mbar.

8. A process according to claim 6, wherein the pressure in the evaporator is 20 to 200 mbar.

9. A process according to claim 1, wherein the compound of formula (1) is isolated by using a batchwise-operating rectifying column, under reduced pressure, to separate less volatile and more volatile by-products therefrom.

10. A process according to claim 1 for the preparation of compounds of formula (1), in which R₁ is tert-butyl, R₂ is methyl or tert-butyl, R₄ is hydrogen and R₃ is methyl, by reacting a compound of formula (2), in which R₁ and R₂ have the given meanings, with a compound of formula (3), in which R₃ and R₄ have the given meanings, in the presence of a base, which process comprises neutralizing the base by adding formic or acetic acid to the reaction mixture after the reaction of a compound of formula (2) with a compound of formula (3) and before the isolation of the compound of formula (1), then filtering the reaction mixture and, to isolate the compound of formula (1) from said reaction mixture, using a rectification apparatus comprising two continuously operating rectifying columns and an evaporator connected upstream, the first of which two columns has a stripping section as well as a rectifying section and the second has a rectifying section only.

11. A process according to claim 10 for the preparation of a compound of formula (1), in which R₁ is tert-butyl, R₂ is methyl, R₄ is hydrogen and R₃ is methyl, by reacting a compound of formula (2), in which R₁ and R₂ have the given meanings, with a compound of formula (3), in which R₃ and R₄ have the given meanings, in the presence of a base, which process comprises adding water to the reaction mixture after the reaction of a compound of formula (2) with a compound of formula (3) and before neutralization.

## Revendications

1. Procédé de préparation de composés de formule 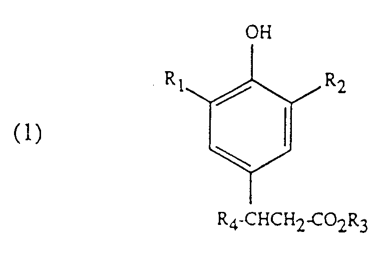 par réaction de composés de formule 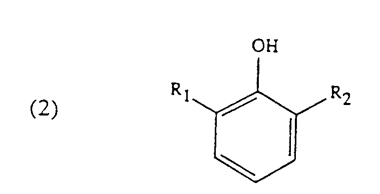 avec des composés de formule
(3) R₄-CH=CH₂-CO₂R₃,
où, dans les formules (1) à (3), les substituants R₁ à R₃ représentent indépendamment les uns des autres des restes alkyle de 1 à 4 atomes de carbone et R₄ est un hydrogène ou un reste alkyle de 1 à 4 atomes de carbone, en présence d'une base, et isolement des composés de formule (1), caractérisé en ce que, après la réaction d'un composé de formule (2) avec un composé de formule (3), et avant l'isolement du composé de formule (1), on ajoute un acide carboxylique au mélange réactionnel pour neutraliser le catalyseur basique, on filtre le mélange réactionnel et on le rectifie pour isoler le composé de formule (1).

2. Procédé selon la revendication 1, caractérisé en ce que l'on utilise de l'acide formique ou acétique pour neutraliser la base.

3. Procédé selon la revendication 1, caractérisé en ce que l'on effectue la neutralisation et la filtration à une température de 50 à 130°C.

4. Procédé selon la revendication 1, caractérisé en ce que, pour isoler le composé de formule (1), on le sépare sous pression réduite des produits secondaires moins volatils et plus volatils à l'aide de deux colonnes de rectification fonctionnant en continu.

5. Procédé selon la revendication 1, caractérisé en ce que, pour isoler le composé de formule (1), on utilise une colonne de rectification fonctionnant en continu munie d'un site de prélèvement latéral et on le sépare au niveau de ce site de prélèvement latéral.

6. Procédé selon l'une des revendications 4 ou 5, caractérisé en ce que l'on dispose, en amont des colonnes de rectification fonctionnant en continu ou de la colonne de rectification fonctionnant en continu munie d'un site de prélèvement latéral, un évaporateur qui fonctionne à une pression plus élevée que les colonnes ou la colonne munie du site de prélèvement latéral.

7. Procédé selon l'une des revendications 4 ou 5, caractérisé en ce que la pression de tête des colonnes de rectification ou de la colonne de rectification munie du site de prélèvement latéral est de 1 à 30 mbars.

8. Procédé selon la revendication 6, caractérisé en ce que la pression dans l'évaporateur est de 20 à 200 mbars.

9. Procédé selon la revendication 1, caractérisé en ce que, pour isoler le composé de formule (1), on le sépare sous pression réduite des produits secondaires moins volatils et plus volatils à l'aide d'une colonne de rectification fonctionnant par charges successives.

10. Procédé selon la revendication 1 pour la préparation de composés de formule (1) dans laquelle R₁ est un t-butyle, R₂ est un méthyle ou un t-butyle, R₄ est l'hydrogène et R₃ est un méthyle, par réaction de composés de formule (2) dans laquelle R₁ et R₂ ont les significations indiquées avec un composé de formule (3) dans laquelle R₃ et R₄ ont les significations indiquées, en présence d'une base, caractérisé en ce que, après la réaction du composé de formule (2) avec le composé de formule (3) et avant l'isolement du composé de formule (1), on ajoute au mélange réactionnel de l'acide formique ou acétique pour neutraliser la base, on filtre le mélange réactionnel et, pour isoler le composé de formule (1) de ce mélange réactionnel, on utilise une installation de rectification qui se compose de deux colonnes de rectification fonctionnant en continu et d'un évaporateur placé en amont, la première des deux colonnes de rectification comportant à la fois une zone d'entraînement et une zone de concentration, la seconde de ces colonnes ne comportant en revanche qu'une zone de concentration.

11. Procédé selon la revendication 10 pour la préparation d'un composé de formule (1) dans laquelle R₁ est un t-butyle, R₂ est un méthyle, R₄ est l'hydrogène et R₃ est un méthyle par réaction d'un composé de formule (2) dans laquelle R₁ et R₂ ont les significations indiquées avec un composé de formule (3) dans laquelle R₃ et R₄ ont les significations indiquées, en présence d'une base, caractérisé en ce que, après la réaction du composé de formule (2) avec le composé de formule (3) et avant la neutralisation, on introduit de l'eau dans le mélange réactionnel.
